# EUROPEAN PATENT APPLICATION

(11) **EP 4 184 524 A1**
(43) Date of publication of application: **24.05.2023**
(21) Application number: 21209500.4
(22) Date of filing: 22.11.2021
(51) Int. Cl.: G16H 50/20

(54) **SYSTEM FOR ASSESSING THE STATE OF A VASCULAR ACCESS**

(71) Applicant: Fresenius Medical Care Deutschland GmbH, 61352 Bad Homburg (DE)
(72) Inventor: NERI, Luca, 61352 Bad Homburg (DE); BELLOCCHIO, Francesco, 61352 Bad Homburg (DE); SCHUMACHER, Erik, 61352 Bad Homburg (DE); BOTLER, Max, 61352 Bad Homburg (DE)
(74) Representative: Bobbert & Partner Patentanwälte PartmbB

(57) **Abstract**

The present invention relates to a system (1) for guiding a clinician in assessing or forecasting the state of a patient's vascular access, comprising a programmable computer (5); an input device (100, 1010, 1030, 1070); an output device (1050), the input device (100, 1010, 1030, 1070) being configured for receiving or collecting input data related to the patient's vascular access; the computer (5) being configured to assess, in an assessment step, the state of the patient's vascular access and/or to provide a prognosis with respect to the patient's vascular access based on the input data; the computer (5) further being configured for outputting the result of said assessment step via the output device (1050).

## Description

The present invention relates to a system according to claim 1 and a method according to claim 15 or according to the preambles or generic terms of each of these claims. A computer program, a digital storage medium and a computer program product are also part of the present invention.

Patients undergoing hemodialysis due to chronic kidney insufficiencies usually require an arteriovenous fistula for vascular access and for providing the required blood flows for the dialysis treatment. Due to the significance of the fistula for the patient's treatment, the fistula needs to be monitored cautiously so that medical issues such as a stenosis can be detected in due course for avoiding adverse health events for the patient.

An object of the present invention is to suggest a system for assessing the state of a patient's vascular access, for monitoring it, and/or for making a forecast or prognosis related to the vascular access, wherein the vascular access can be an arteriovenous fistula without being limited thereto.

Moreover, a method therefor should be suggested. In addition, a computer program, a digital storage medium and a computer program product should be suggested.

The objective of the present invention is achieved by the system having the features of claim 1. It is further achieved by the method according to claim 15, the computer program, the digital storage medium and the computer program product being part of the present invention.

According to the present invention, a system for assessing or for guiding or assisting a clinician in monitoring, assessing and/or predicting the state and/or making a prognosis of a patient's vascular access is suggested.

The system comprises a programmable computer, at least one input device and at least one output device.

Thereby, the input device is configured for receiving or collecting input data related to the patient's vascular access.

The computer is configured to receive, in a signal communication or communication connection, the input data from the input device.

Furthermore, the computer is configured to assess and/or predict, in an assessment step, the state of the patient's vascular access, based on the input data. Alternatively or additionally, the computer is configured to provide a prognosis with respect to the patient's vascular access based on the input data.

The computer is further configured for outputting the result of said assessment step via the output device.

The method according to the present invention for guiding or assisting a clinician in monitoring, assessing and/or predicting the state and/or making a prognosis of a patient's vascular access comprises the following steps:
- providing a system according to the present invention;
- receiving or collecting input data related to the patient's vascular access
- inputting said data into the computer via the input device;
- assessing, using the computer in an assessment step, the state of the patient's vascular access and/or providing a prognosis with respect to the patient's vascular access based on the input data;
- outputting, via the computer, the result of said assessment step via the output device.

According to the present invention, a computer program with a program code is suggested, programmed such that a system comprising a programmable computer, an input device and an output device is re-programmed into a system according to the present invention.

According to the present invention, a computer program can, for example be taken to mean a physical, distributable software-product, which comprises a program. A computer program according to the present invention comprises a program code, so that a conventional computer system can be programmed to be a computer system according to the present invention if the computer program is running on a corresponding computer.

A digital, particularly non-volatile storage medium, according to the present invention, particularly in the form of a machine readable carrier, particularly in the form of a diskette, CD, DVD EPROM, FRAM (Ferroelectric RAM) or SSD (Solid-State-Drive), particularly with electronically or optically readable control signals, is designed in such a way as to interact with a system comprising a programmable computer, an input device and an output device so that a conventional system is reprogrammed into a system according to the invention when its memory content runs on the programmable system.

A computer program product, according to the present invention, comprises a volatile or transient program code or one stored on a machine readable carrier or a signal wave, configured to interact in such a way with a programmable computer configuration of a system comprising a programmable computer, an input device and an output device so that the system can be reprogrammed to be a system according to the present invention.

Computer program product, for example, can be understood according to the present invention as a computer program stored on a medium, an embedded system being a comprehensive system with a computer program (e.g., an electronic device with a computer program), a network of computer implemented computer programs (e.g. client/server-system, a cloud computing system etc.), or a computer on which a computer program is loaded, runs, is stored, is being performed or developed.

The term "machine readable carrier" as used herein, refers in certain embodiments of the present invention to a carrier, which contains data or information interpretable by software and/or hardware. The carrier may be a data carrier, such as a diskette, a CD, DVD, a USB stick, a flashcard, an SD card or the like, as well as any other storage referred to herein or any other storage medium referred to herein.

According to the present invention, a computer program product may be understood as a programmed application (in short: app), for example, particularly for a smartphone, a tablet or another mobile hand-held device.

Embodiments according to the present invention may comprise one or several of the features mentioned supra and/or in the following in any combination unless the person skilled in the art considers the particular combination to be technically impossible.

Advantageous developments of the present invention are each subject-matter of the dependent claims and embodiments.

In all of the aforementioned and following statements, the use of the expression "may be" or "may have" and so on, is to be understood synonymously with "preferably is" or "preferably has", and so on respectively, and is intended to illustrate an embodiment according to the present invention.

Whenever alternatives with "and/or" are introduced herein, the person skilled in the art will understand the "or" contained therein as preferably "either or" and preferably not as "and".

Whenever numerical words are mentioned herein, the person skilled in the art shall recognize or understand them as indications of numerical lower limits. Hence, unless this leads to a contradiction evident for the person skilled in the art, the person skilled in the art shall comprehend for example "one" (or "a/an") as encompassing "at least one". This understanding is also equally encompassed by the present invention as the interpretation that a numerical word, for example, "one" (or "a/an") may alternatively mean "exactly one", wherever this is evidently technically possible in the view of the person skilled in the art. Both of these understandings are encompassed by the present invention and apply herein to all used numerical words.

In case of doubt, the person skilled in the art will understand spatial information like "above", "below", "upper", "lower", "left" or "right" whenever they are herein mentioned, as a spatial indication with reference to the alignment in the figures appended hereto and/or of the arrangement of the system according to the present invention when used as intended. "Bottom" is closer to the centre of the earth or to the bottom of the figure than "top".

Whenever an embodiment is mentioned herein, it represents an exemplary embodiment according to the present invention.

When it is disclosed herein that the subject-matter according to the present invention comprises one or several features in a certain embodiment, it is also respectively disclosed herein that the subject-matter according to the present invention does, in other embodiments, likewise according to the present invention, explicitly not comprise this or these features, for example, in the sense of a disclaimer. Therefore, for every embodiment mentioned herein it applies that the converse embodiment, e.g. formulated as negation, is also disclosed.

When method steps are mentioned herein, then the system according to the present invention is in several embodiments configured in order to execute, in any combination, one or several of these method steps, in particular when these are automatically executable steps, or in order to accordingly control corresponding apparatuses which preferably correspond with their names to the designation of the respective method step (for example, "determining" as a method step and "apparatus for determining" for the apparatus) and which may likewise be part of the apparatus(es) according to the present invention or connected thereto in signal communication. Also, in some embodiments, the method according to the present invention encompasses the steps described herein with respect to the device, in particular with respect to the programming or usability of the system or its devices.

When "programmed" or "configured" is mentioned herein, then these terms may in some embodiments be interchangeable.

When a signal communication or communication connection between two components is mentioned herein, this may be understood to mean a connection that exits during use. It may also be understood that a preparation for such a (wired, wireless or otherwise implemented) signal communication exists, for example by coupling both components, for example by pairing, etc.

Pairing is a process that takes place in connection with computer networks in order to establish an initial link between computer units for the purpose of communication. The best-known example of this is the establishing of a Bluetooth connection, by which various devices (e.g. smartphone, headphones) are connected to each other. Pairing is sometimes also referred to as bonding.

When method steps are mentioned herein, then it is provided in several embodiments according to the present invention that these steps are carried out prior to or after a treatment of the patient, for instance during the time when the patient is not connected, e.g. via the extracorporeal blood circuit or the like, to the apparatus or that a treatment has not begun yet or is already completed.

The programmable computer may prompt the execution of all or substantially all of the method steps. The method according to the present invention may substantially or completely be executed by the computer. It may be partly executed by the computer.

Whenever "clinician" is mentioned herein, this term comprises nurses, doctors, clinical or caring staff.

In some embodiments, the patient's vascular access is the shunt or the native vessel used for withdrawing blood from the patient's blood system when conducting an extracorporeal blood treatment in a blood treatment session.

In some embodiments of the system according to the present invention, the computer is further configured to assess the state of the patient's vascular access in a corresponding assessment step. Alternatively or additionally, the computer is configured to provide a prognosis with respect to the patient's vascular access based on at least one predefined criterion.

The criterion may be, e. g. a comparison of the input data with corresponding data of further patients, earlier measurements (development) with respect to the same patient, commonly used medical scores, to thresholds, e. g. ones set by the clinician, and the like.

In several embodiments, the input data is or comprises patient examination or diagnosis data, in particular longitudinal patient data, in particular with respect to the patient's vascular access, physiological patient data, and/or medical device data.

In some embodiments, the examination or diagnosis data is selected from the group consisting of or comprising images of the vascular access (MRI (magnet resonance imaging), CT (computer tomography), photos, e.g., that may be compared to photos taken at an earlier visit of the patient in the clinic, and the like), audios of the vascular access, pulse wave data from pulse-wave imaging based on ultrasound elasticity imaging for the spatial-temporal mapping of the pulse wave-induced arterial wall motion, one or more assessments by clinicians, and the patient's report.

In several embodiments, the longitudinal patient data is or comprises historic patient data, e.g. drugs and/or treatments administered to the patient, and/or medical intervention performed, like examinations, surgeries or the like.

In some embodiments, the physiological patient data is or comprises patient-specific data, such as age, sex, weight, body mass index, blood pressure, and or ECG (electrocardiogram) data or the like. Preferably, the physiological patient data is or comprises no pathological data.

In several embodiments, the medical device data describes an aspect of a medical treatment device's operation or relates thereto. This may be or comprise data attained or measured with respect to the operation of the medical device, particularly during dialysis treatment, for example, arterial/venous pressure, ultrafiltration volume, clearance, number of alarms etc.

The data measured during dialysis treatment may be data measured or attained during an ongoing dialysis treatment session, i.e., while the computer is assessing or predicting, in the assessment step, the state of the patient's vascular access and, hence, during an ongoing treatment session. Such data may, however, alternatively have been measured during a past and, hence, already terminated dialysis treatment session.

In some embodiments, the computer is configured to assess and/or predict the input data, in the assessment step, preferably based on advanced analytics.

The term "advanced analytics" may comprise the semi-autonomous or autonomous analyses of data. Thereby, sophisticated methods and tools from the state of the art, e.g. regression, clustering, cohort analyses, recommendation algorithms, optimization, in particular Bayesian network optimization, gradient boosting (e.g. the bibliotheque XGBoost), (decision) tree algorithms, preferably directed and/or uncyclic, influence diagrams, probabilistic graphical models and the like may be used.

In several embodiments, "advanced analytics" may in particular comprise methods which make it possible, on the basis of empirically determined data, to derive models that allow assessments and predictions on that database.

Alternatively or additionally, different methods of Data Mining may in some embodiments, for example, be comprised under the term "advanced analytics".

In several embodiments, the advanced analytics comprise algorithms based on neural networks and/or machine learning, in particular deep learning and/or reinforcement learning.

Neural-network architectures can include convolutional neural networks, long-short-term memory networks, recurrent neural networks or other types of neural networks, which are usually employed for optimization or classification problems known in the state of the art.

In some embodiments, the computer is configured to allot a diagnostic and/or prognostic value to the input data in the assessment step. The input data may be or comprise, for example, the findings of an examination or diagnosis of the particular patient, individual physiological patient data, and/or medical device data relating to the treatment of the particular patient. Alternatively, the computer is configured to allot a diagnostic and/or prognostic value, likewise in the assessment step, to the input data in its entirety.

The diagnostic and/or prognostic value may be a probability of a particular outcome such as an adverse health event, in particular with respect to the vascular access, a risk score, an objective medical score, and so on. Also, in addition or alternatively, the diagnostic and/or prognostic value may indicate a severity, an urgency, or the like.

For example, an image taken of the vascular access may be allotted 3 points out of 5 possible point, 5 points indicating that the vascular access is in perfect shape and 0 point indicating that the vascular state is in the worst state imaginable. The number of points can be set by, e.g., an algorithm trained based on a sufficient number of images.

Also, again by way of example, the clinician assessing the vascular access using her bare eye, or palpating the vascular access, e.g. an arteriovenous fistula, can also attribute a number of points up to a maximum number of, e.g., 5 possible points.

Similarly, a corresponding value may be attributed to any other of the data input to the input device.

Alternatively, or in addition, one value may be attributed to the input in general. That value may be comprised by an algorithm that takes several values (one, e.g., for the clinician's assessment, one, e.g., for the analysis of an image taken, and so on) into account, or it may be gained in a different way while contemplating all available input data.

In several embodiments, the computer is programmed or configured to consider, in the assessment step, the medical resources or devices available for further assessing the vascular access, in particular the arteriovenous fistula.

In particular, the medical resources or devices available may be those which are at the patient's or the clinician's disposable. Hence, the term medical resources or devices may denote those medical resources or devices that can be used in a given setting, e.g. at the hospital to which the patient is addressed for undergoing blood treatment, that can be handled by the clinicians being present at that time and/or that can be used for the particular patient regarding her medical insurance status, or the like. The available medical resources or devices may, for example, be listed in a storage device accessible by the computer. The storage device may also comprise information regarding further aspects of interest with regard to additional features of the medical resources or devices available, such as the one disclosed in this paragraph or anywhere else within the present specification. In some embodiments the storage device may be part of the system according to the present invention.

In several embodiments, the computer is configured to allot, or attribute, in the assessment step, a benefit value to the individual medical resources or devices available, in particular with respect to the input or the diagnostic and/or prognostic value(s).

Hence, the benefit value of an individual medical resource or device may have been determined in relation to a given diagnostic and/or prognostic value before. For example, based on experiments or testing, it may have been found that the status of the vascular access that has been attributed 3 points out of 5 by the clinician based on a visual diagnosis can best be further assessed by a pulse-wave imaging method based on ultrasound elasticity imaging. Additionally or alternatively, it might be known, again from experiments, that starting from a 3 points out of 5 in the visual assessment by a clinician, it is of little help, in further assessing the real status of the vascular access, to ask the patient to undergo a MRI or CT assessment for gaining images of the vascular access. Hence, the benefit value of any medical resource or device may depend on the particular diagnostic and/or prognostic value gained from the input available. In particular, the benefit value of any medical resource or device may depend not only on one particular diagnostic and/or prognostic value but on a multitude of diagnostic and/or prognostic values available (such as 3 points out of 5 in the visual assessment, and 4 points out of 5 in the assessment of the physiological patient data at the same time, or the like). Hence, the benefit value of each medical resource or device may depend on the combination of input values and/or their particular specification, height, parameter value, and so on.

Also, in some embodiments, the benefit value of any medical resource or device may depend not only on diagnostic and/or prognostic values but also on additional medical resources or devices that may be available at the same time. Hence, based on a given input value, the benefit value of a particular medical resource or device (a pulse-wave imaging method, for example) may depend on the availability of another particular medical resource or device (a CT image, for example). In other words, the benefit value of a first medical resource or device can be higher if a particular second medical resource or device is available as well when compared to a situation in which only the first medical resource or device is available while the second one is not. In other words, interdependencies and reciprocal effects may be reflected in the benefit value, depending in some embodiments, however, on the underlying input value.

It goes without mentioning that the benefit value may also reflect economic aspects involved in resorting to the particular individual medical resource or device or combinations thereof. In particular, the benefit may be lower the more expensive the medical resource or device is and/or the more medical resources or devices have been combined in order to achieve a desired information when using it.

Also, in certain embodiments the benefit value can be linked to a particular point in time or reflect the same. For example, a particular medical resource or device may be of higher benefit if available still today but of lower benefit if not available until next week.

In some embodiments, the benefit value may indicate a probability of gaining a missing, particular information regarding the state of the vascular access, or a probability of improving the state of a vascular access, based on the given input value.

It is noted that, like the diagnostic and/or prognostic value, the benefit value may be a qualitative or a quantitative value.

In some embodiments, the computer comprises and/or is programmed or configured to use, in the assessment step, a utility maximization layer and/or utility maximization function.

A utility maximization layer or function can be understood in the present context as an algorithm outputting a suggestion to the clinician in charge based on the input for the utility maximization layer or function as set forth herein. It may base on or comprise functions and methods known from the state of the art, in particular as pointed out herein under the explanation of the term "advanced analytics".

That layer or function can be programmed or arranged to suggesting an optimal medical action to the clinician. The optimal medical action can be calculated based on the input, preferably weighted by individual weighting factors. For example, a suggestion may be considered optimal once it cannot be amended in the light of the analysed input.

The combination of goods or services that maximize utility is determined by comparing the marginal utility of two choices and finding the alternative with the highest total utility, for example within a budget limit. For example, the decision can be influenced by the option that produces a higher level of satisfaction.

In several embodiments, the output result is or comprises one or more medical actions to be taken or recommendations for medical actions. Medical action may, for example, be or comprise a particular medical examination, a medical treatment, a medical intervention such as surgical intervention, and the like. In certain embodiments it may be additionally provided that the medical actions are ranked.

In some embodiments, the computer is configured to output the result of the assessment step (as described herein) on the output device, wherein the output may comprise both the diagnostic and/or prognostic value(s) allotted to the input data and the benefit value allotted to the individual medical resources or devices available.

In several embodiments, the output device is configured for outputting the result of the assessment step received via wired or wireless means, in particular via Ethernet, optical fibers, Wi-Fi, LTE or 5G, in particular to a clinician's smartphone, tablet, or personal computer [using, e.g. graphical user interface].

In some embodiments, the computer is configured to repeatedly carry out the assessment step or sub-steps thereof, in particular automatically. This may be particularly advantageous if, when or each time additional input data related to the patient's vascular access is received or collected by the input device. The repetition may result, e.g., in refreshed or recalculated diagnostic, prognostic and/or benefit values.

In several embodiments, the system according to the present invention further comprises a medical treatment apparatus. The medical treatment apparatus may be in (mutual) signal communication with the input device or the computer, or prepared therefor. Additionally, the computer is programmed to use data provided by the medical treatment apparatus in the assessment step.

In some embodiments, the system further comprises at least one medical examination device. The medical examination device may be in (mutual) signal communication with the input device or the computer, or prepared therefor. Additionally, the computer is programmed to use data provided by the medical examination device in the assessment step.

In several embodiments, the system further comprises an alarm device for signalling or emitting an alarm.

Thereby, the computer is configured to output an alarm via the alarm device if the presence of pre-determined conditions is detected in the assessment step.

Such a pre-determined condition may be or comprise, for example, that an objective quantitative and/or qualitative medical score (as further explained below) exceeds or undercuts some predetermined value within the assessment step. This may, e.g., be the case if
- an aneurism is predicted to occur with x % in the next few weeks;
- the utility maximization layer proposes a surgical intervention asap/within a time frame; or the like.

In certain embodiments the alarm may be accompanied by an intervention proposal via the utility maximization layer.

In some embodiments, the output data generated by the prognostic layer, i.e., the diagnostic or prognostic values can include health information such as a probability or a risk score related to the occurrence of an adverse health event and/or recommended medical actions. The diagnostic or prognostic values can further comprise objective medical data derived from the input data, e.g., indicative to blood flow characteristics like the average flow velocity and/or the peak flow velocity within the arteriovenous fistula.

In certain embodiments, the diagnostic or prognostic values can provide health information regarding the state of the vascular access derived from, for example, an image or video of the vascular access or the surrounding body area, the computer program can compute a probability or a risk value related to developing an aneurism and/or can categorize the vascular access based on its optical appearance by assigning an objective medical score, which can be related to a medical risk or the occurrence of an adverse health event, e.g., for developing an aneurism. For example, the objective medical score can be related with a specific probability based on a lookup table. Further output data within the prognostic layer can comprise a probability or risk score associated with the development of an arteriovenous stenosis, e.g., the existence of a stenosis, a stenosis degree (based on classifications such as the ECST (European Carotid Surgery Trial) or the NASCET (North American Symptomatic Carotid Endarterectomy Trial)) or a failure probability within a predefined time period. The stenosis risk score can be based on audio data, longitudinal patient data, pulse-wave data, and/or medical device data as input data for the computer program.

As set forth supra, the diagnostic or prognostic values can rely on all possible input data or on a sub-set of all possible input data. In the case that more input data becomes available eventually, the diagnostic or prognostic values can be updated. For example, in the case that only audio data from a digital stethoscope and longitudinal patient data is available in the first place, preliminary diagnostic or prognostic values can be provided. As soon as additional information become available, e.g., a video of the vascular access is included to the input data, the diagnostic or prognostic values can be updated or stretched accordingly. For instance, risk scores and/or probabilities for health events can be adjusted and provided to the user and/or the utility maximization layer of the computer. The adjusted risk scores and probabilities can also be provided with an accuracy information comparing, e.g., the situation before and/or after the additional information has become available.

The utility maximization function can be a routine of the computer or computer program already comprising the prognostic layer, or a separate computer or computer program located on a local or remote computing device also forming part of the system according to the invention.

The utility maximization function can be based on decision-tree algorithms, deep neural networks, and or other types of optimization algorithms.

In some embodiments, the utility maximization function is being trained or was trained using data of historic patient histories, such data having been available from suitable data bases to train the utility maximization function model, e.g., a deep-learning model.

In certain embodiments, reinforcement learning based on, e.g., a Q-learning algorithm, is being employed or was employed for modelling the prognostic layer and/or the utility maximization function. In this case, reinforcement learning allows to automatically optimize a number indicative for the quality of the model over time, e.g., the recommendations for medical actions or the benefit values and/or the diagnostic or prognostic values, provided the utility maximization function and/or the prognostic layer are adjusted such that a number is optimized. Such numbers can include or be related to the maximization of patient lifetime or the optimization of other objective health-related numbers, e.g., the minimization of the occurrence of life-threating conditions like strokes or heart attacks within a given time period.

Output data can further be sent to other computer programs and/or provided to clinicians such as nurses or doctors. In one example, the output data can be sent via wired and/or wireless means such as Ethernet, optical fibers, Wi-Fi, LTE or 5G, and displayed on the clinician's smartphone, tablet or personal computer using, e.g., a graphical user interface. An alarm can also be provided to a clinician, e.g., triggering visual and/or acoustical signals, if certain medical scores, e.g., the NASCET classifications, exceed or undercut predefined threshold limits.

In some embodiments it is the computer or one of its hard- or software components such as the utility maximization layer that takes a decision as to how to proceed based on the suggested output. For example, it may be decided by the system with which medical resources or devices the state of the vascular access should be examined next based, e. g. on the list generated by the utility layer. In particular, the output may consist of or comprise commands sent to a machine, a monitor, staff and so on to get a particular medical resource or device ready for continuing the evaluation of the patient"s vascular access.

In certain embodiments the system encompasses all medical resources and devices available in the particular clinic where the patient's vascular access is being assessed and will further be evaluated if required. Hence, in some embodiments, the system may comprise all machines that may be turned to based on the list of the suggested machines generated by the utility maximization layer. Also, in certain embodiments the system encompasses all medical examination devices available in the particular clinic that might contribute input data to the system for assessing the vascular access" state such as machines for taking images of the vascular access, audios of the vascular access, pulse wave data from pulse-wave imaging based on ultrasound elasticity imaging for the spatial-temporal mapping of the pulse wave-induced arterial wall motion, and so on.

In some embodiments, the system is the clinic or hospital where the patient's vascular access is being assessed and will further be evaluated if required.

In certain embodiments, the invention's purpose is to provide guidance or information for a clinician in assessing the state of the patient's vascular access as, e. g., set forth herein.

In some embodiments, the invention's purpose is to define the next step in assessing the state of the patient's vascular access, e. g. by the output of the computer or, for example, the utility maximization layer as, e. g., set forth herein.

In certain embodiments, the invention's purpose is to assess or further assess the state of the patient's vascular access as, e. g., set forth herein.

In some embodiments the invention encompasses the medical steps of gaining the input data, such as measuring, taking pictures, and the like, by means of the medical examination devices that actually contribute data to the computer. In other embodiments these steps are not encompassed or comprised by the invention.

In some embodiments the system, in particular its computer carries the steps discussed herein automatically. Similarly, the method steps can be automatic ones.

Some or all of the embodiments according to the present invention may comprise one, several or all of the advantages mentioned above and/or in the following.

In practice, medical examinations such as radiographic or sonographic examinations depend on various resources including personnel, energy, time, and further economic costs. In addition, it is a non-trivial task to determine the best examination for a given patient at a given point in time, which takes into account various constraints such as the audio-visual state of the vascular access, the overall health state of the patient, the treatment state, or the present disease progression. Nevertheless, making good medical decisions so that correct examinations are timely performed is crucial for preventing adverse health outcomes for patients. The present invention allows to guide the clinician in charge through analysis and intervention steps. Also, interdependencies between different diagnostic or prognostic states that may not simultaneously be contemplated by a human may be observed by the present invention.

Hence, the output suggestion from the utility maximization layer may, and practically in all cases will, because of, e.g., the complexity of the considerations resulting in the output, be accepted by the clinician without hesitation.

In some embodiments, the present invention assists in optimizing the use of the available medical resources and devices in the setting of an entire hospital when it comes to further assessing dialysis patients" vascular accesses. Examinations that are of second-rate use for the particular patient's particular needs may remain disregarded whereas the optimal fit is being gone for. That way, the technical effort and apparatuses provided or maintained at a hospital for assessing patient's vascular accesses can be reduced to the appropriate level.

Another advantage provided by some embodiments may be observed in the assistance which the system may provide to the clinician in choosing the best fitting medical resources or devices to further assess the vascular access" state.

The diagnostic or prognostic values may further allow to determine suitable points of time for calling in the patient for another assessment or examination of her vascular access" state. For example, the diagnostic or prognostic value may, in particular, be compared to earlier findings for the same patient, in order to determine the time which should not be exceeded for calling in the patient for another check of the vascular access" state. Hence, in some embodiments, the computer is configured to mark, book, state or otherwise identify a day, a month, a time (coming November, next year, calendar week 41, and the like) for the next check. In certain embodiments, the computer of the system is configured to anchor this information beyond or outside the present system. This may happen by sending out an invitation to the clinician, and/or patient indicating a suitable time for her next vascular access check, by placing an entry in the patient's medical record, by controlling a printer to print out a suitable note to be taken to the medical record, by sending out messages or reminders to the clinician and/or to the patient, e. g. via smartphone, email, letter and so on, and/or to interact with an environment outside the system in any other suitable way.

Another advantage provided by the present invention may be the integration of expert knowledge into available data in order to achieve the best assessment results in a given situation regarding the state and/or prognosis of a patient's vascular access.

All advantages achievable with the method according to the present invention can be in certain embodiments achieved undiminished by the system and devices according to the present invention and vice versa.

Below, the present invention is described based on preferred embodiments thereof with reference to accompanying drawing. However, the present invention is not to be limited to these embodiments. In the figures, in which same reference numerals denote identical or similar elements, values and so on, the following applies:
- **Fig.** 1: shows schematically simplified a system according to the present invention for assessing the state and/or prognosis of a patient's vascular access in an exemplary embodiment; and
- **Fig.** 2: shows schematically simplified a fluid line structure of a blood treatment apparatus of the system in an exemplary embodiment.

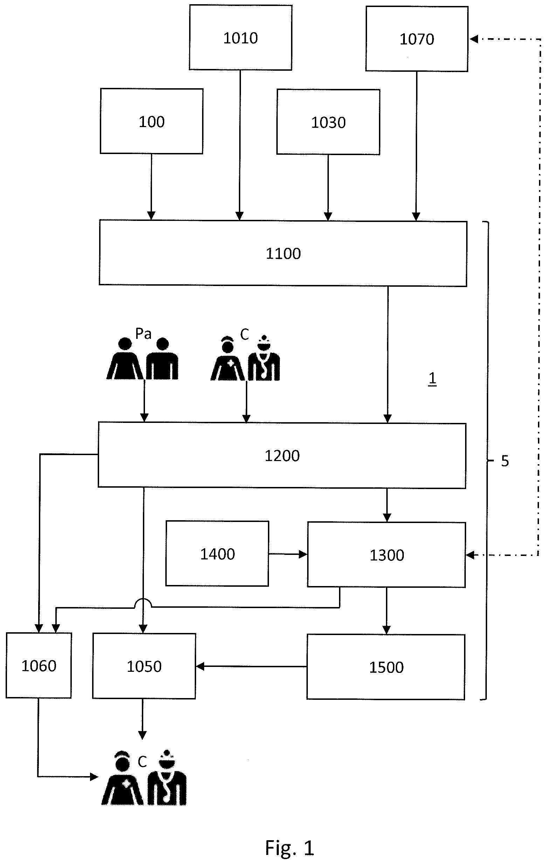

**Fig.** 1 shows schematically simplified a system 1 according to the present invention for monitoring, assessing and/or predicting the state and/or prognosis of a patient's vascular access in an exemplary embodiment.

In the example of Fig. 1 the system 1 comprises a programmable computer 5, at least one input device, exemplary denoted by the reference numerals 100, 1010, 1030,1070 discussed infra, and an output device 1050.

The input device may be configured for receiving or collecting input data related to the patient's vascular access from, e.g., a medical treatment apparatus 100 and/or from at least one medical examination device 1010 and/or from a storage device 1070 and/or from the device 1030 for additional input.

The input device may be comprised by one, some or all of the above mentioned devices, i.e., the medical treatment apparatus 100, the at least one medical examination device 1010, the storage device 1070 or the device 1030 for additional input, in any combination. The input device may include additional devices which are known to one skilled in the art, even though they are not explicitly described herein.

The computer 5 is configured to assess and/or predict, in an assessment step, the state of the patient's vascular access and/or to provide a prognosis with respect to the patient's vascular access based on the input data.

For this purpose, on the computer 5 there may run a computer program 1100 consisting of or comprising algorithms, preferably based on advanced analytics, which are able to take into consideration, in the assessment step, the input data entered via the device 1030 for additional input, input data retrieved from a medical treatment apparatus 100, from at least one medical examination device 1010 and/or from a storage device 1070.

Therefore, the computer 5 may be in signal communication with the device 1030 for additional input, the medical treatment apparatus 100, the medical examination device 1010 and/or the storage device 1070. At least it is prepared therefor.

Hence, the computer program 1100 may analyse the input received from the input device or its individual sources 100, 1010, 1030 or 1070. The computer 5 may be configured to, in the assessment step, do so by providing a prognosis with respect to the patient's vascular access, for example based on at least one predefined criterion, e.g. by comparing the input data to corresponding data of further patients, earlier measurements (development) with respect to the same patient, commonly used medical scores, to thresholds, e.g. ones set by the clinician, and the like.

The input data, i.e. the examination or diagnosis data, may be selected from the group consisting of or comprising images of the vascular access, audios of the vascular access, pulse wave data from pulse-wave imaging based on ultrasound elasticity imaging for the spatial-temporal mapping of the pulse wave-induced arterial wall motion, at least a clinician's assessment and the patient's report.

The longitudinal patient data is or comprises historic patient data, e.g., administered drugs, administered treatments, and/or performed medical intervention like examinations or surgeries or the like.

The physiological patient data is or comprises patient-specific data, e.g., age, sex, weight, body mass index, blood pressure, and or ECG data. Preferably, it comprises no pathological data.

The medical device data describes aspects of a medical device's operation and is or comprises data attained or measured with respect to the operation of the medical device during dialysis treatment, e.g., arterial/venous pressure, ultrafiltration volume, clearance, number of alarms, in particular pressure alarms, etc. The medical device data may in some embodiments be or comprise data retrieved from the medical treatment apparatus 100 and/or from the medical examination device 1010.

Further data retrieved from input made by the patient Pa or any clinician C who is responsible for the patient Pa and/or their treatment may be added to the data retrieved by the computer 5 and pre-processed or further processed by the computer program 1100 consisting of or comprising algorithms based on advanced analytics. Relevant data at this point may be, for example, outcomes reported by the Patient Pa and/or the risk assessment by a clinician C.

In consequence, a probability of a particular outcome such as an adverse health event, in particular with respect to the vascular access, a risk score, an objective medical score, and so on may be computed, retrieved or otherwise obtained. The corresponding information or result of the assessment step is herein also addressed as diagnostic and/or prognostic value(s), and it may, e.g., reflect the personalized risk of a failure of the patient's vascular access.

The diagnostic and/or prognostic value(s), taken as input for the modular prognostic layer 1200 as set forth above may already allow making an assessment or prognosis concerning the vascular access of the patient Pa. In addition, the modular prognostic layer 1200 may receive and process in certain embodiments assessments or risk assessments of the clinician C in charge or another person, also by outcomes or observations reported by the patient Pa.

The diagnostic and/or prognostic value(s) generated by the modular prognostic layer 1200 as described herein are input into a utility maximization function 1300 which is provided in order to supplement, complete, further process etc. the data.

Also, the utility maximization function 1300 may take into consideration further data 1400, in particular benefit values related to medical devices and resources as disclosed supra, the availability of said medical devices and resources, such as a particular medical examination, a medical treatment, a medical intervention such as surgical intervention, and the like, i.e. the availability of resources and devices, their cost-effectiveness, the probability of gaining a missing, particular information regarding the state of the vascular access, a probability of improving the vascular access state, an economic aspect involved in resorting to the particular individual resource, and so on, and combinations thereof. Also, the benefit value can be linked to a particular point of time. The device in which the further data 1400 are stored is advantageously accessible by the computer 5 and may be part of the system 1.

Based on the data available to the utility maximization function 1300, and in particular the benefit values, a list of suitable medical devices and resources for taking care or for further investigating or examining the vascular access may be generated by the utility maximization function 1300 for being presented to the clinician.

Also, the list may comprise suitable medical devices and resources in a suitable or suggested order. Hence, a ranking may be presented. For example, the ranking may be triggered by the benefit values in decreasing order.

This leads to a ranking or recommendation of a medical action that may be kept and/or stored in a utility maximization layer 1500.

The computer 5 is further configured for outputting the result of the assessment step of the utility maximization layer 1500 via the output device 1050 in order to be noted by a responsible clinician C. The clinician C may or may not take the necessary medical step(s) from the ranking, due to (depending on) their knowledge or experience. The output result may comprise both the diagnostic and/or prognostic value(s) allotted to the input data and the benefit values allotted to the individual medical resources or devices available.

The control device of the system 1 may be programmed to output or display, via the output device 1050, the results of the assessment step received via wired or wireless means, in particular via Ethernet, optical fibers, Wi-Fi, LTE or 5G, in particular to or on a receiving device such as a monitor or a graphical user interface, a clinician's smartphone, tablet, or personal computer.

It is noted that the system 1 may comprise and use algorithms based on neural networks and/or deep learning or machine learning to come to the results of the modular prognostic layer 1200 or the utility maximum layer 1500.

In the system 1 according to the present invention the computer 5 may be configured to repeatedly carry out the assessment step or sub-steps thereof, e.g., refreshing or recalculating the diagnostic and/or prognostic value and/or the benefit value, in particular automatically, in particular if, when or each time additional input data related to the patient's vascular access is received or collected by the input device 1030 or any other device which is suitable to provide input as described above.

The system 1 may further comprise an alarm device 1060 for signalling or emitting an alarm, in particular when the presence of pre-determined conditions, in particular those affecting the patient's health or the success of the treatment, is detected. This detecting may particularly happen either within the modular prognostic layer 1200 or the utility maximization layer 1500.

As explained herein, the utility maximization layer 1500 provides output data based on the results as received from the utility maximization function 1300. Output data within the utility maximization layer 1500 can comprise medical actions, which are recommended to be performed based on input data and available resources. Medical actions can comprise appropriate medical examinations, medical treatments and/or other medical interventions like surgical interventions. The medical actions can be ranked so that most efficient medical actions receive a higher-ranking number than less efficient ones, wherein efficient medical actions can be characterized as actions which provide the best medical outcome for one or a cohort of patients, e.g., the highest survival probability within a given time frame, in view of the required resources, e.g., personnel, time, energy, economic resources such as costs, for a medical action or intervention. For example, sonographic or angiographic examinations can be expensive and time-consuming so that only a fraction of all patients having a vascular access can be examined within a given time period. Based on the provided input data, the output data provided in the prognostic layer 1200 are considered together with available resources, i.e., the utility maximization function 1300 can also query clinical databases for available resources, to recommend the best medical-action strategy for individual patients. Such databases can be comprised by the system 1. For example, elderly patients having a high bodymass index with clear indications for a stenosis due to a noticeable murmur pattern from a digital stethoscope examination are much more likely referred for further examinations than, e.g., younger patients without any evidence for a stenosis.

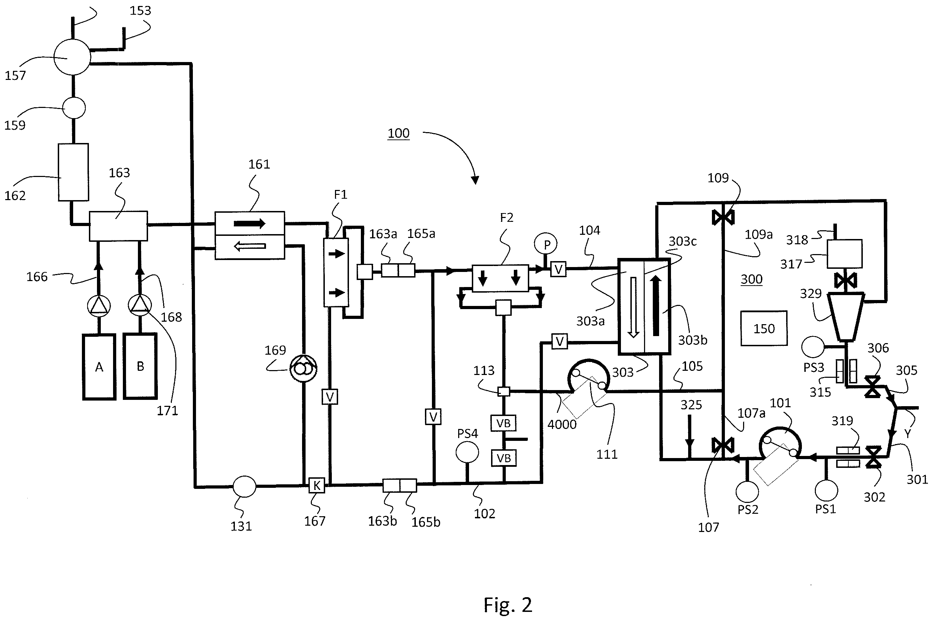

**Fig. 2** shows schematically simplified fluid line structure of a blood treatment apparatus 100 in a first, purely exemplary embodiment of the system 1.

The blood treatment apparatus 100 is connected to an extracorporeal blood circuit 300, which can be connected to the vascular system of the patient, not shown, for a treatment using double-needle access, or via single-needle access using, for example, an additional Y-connector (reference numeral Y) as shown in Fig. 2. The blood circuit 300 may be present, optionally in sections, in or on a blood cassette.

The system 1 can guide a clinician C in assessing the state of the vascular access (not shown), when the extracorporeal blood circuit 300 is connected to it or is to be connected or was connected to it.

Pumps, actuators and/or valves, for example, in the area of the blood circuit 300 are connected in signal communication, or prepared therefor, with the blood treatment apparatus 100 or to a control device 150 (which optionally may be a closed-loop control device) optionally encompassed by it.

The control device 150 may effect an output of data regarding the blood treatment apparatus 100 which can be used in the system 1 according to the present invention as input data to the programmable computer 5 in order to assess the state of a patient's vascular access.

The blood circuit 300 comprises or is connected to an arterial patient tubing clamp 302 on an arterial section or an arterial patient line, blood withdrawal line or arterial blood line 301, here connected to an arterial connection needle. The blood circuit 300 further comprises or is connected to a venous patient tubing clamp 306 on a venous section or a venous patient line, blood return line or venous line 305, here connected to a venous connection needle.

A blood pump 101 is provided in or at the arterial blood line 301, a substitute fluid pump 111 is connected to a dialysis liquid inlet line 104 for conveying fresh dialysis liquid, which is filtered in a further filter stage (F2) (substitute fluid). A substitute fluid line 105 may be fluidically connected to the inlet line 104. Using the substitute fluid pump 111, substitute fluid may be introduced by pre-dilution, via a pre-dilution valve 107, or by post-dilution, via a post-dilution valve 109, via associated lines 107a or 109a into line sections, for example into the arterial blood line 301 or into the venous blood line 305 (here between a blood chamber 303b of a blood filter 303 and a venous air separation chamber or venous blood chamber 329) of the blood circuit 300.

The blood filter 303 comprises the blood chamber 303b connected to the arterial blood line 301 and to the venous blood line 305. A dialysis liquid chamber 303a of the blood filter 303 is connected to the dialysis liquid inlet line 104 which leads to the dialysis liquid chamber 303a and to a dialysate outlet line 102 which leads away from the dialysis liquid chamber 303a, which conveys dialysate, i.e. used dialysis liquid. For this purpose, suitable connectors are used on the dialysis liquid inlet line 104 or on the dialysate outlet line 102 on the one hand and on the dialysate ports on the other hand, which can be connected to one another, in particular in a detachable manner.

Dialysis liquid chamber 303a and blood chamber 303b are separated from each other by a mostly semi-permeable membrane 303c. It represents the partition between the blood side with the extracorporeal blood circuit 300 and the machine side with the dialysis liquid or dialysate circuit, which is shown in Fig. 2 to the left of the membrane 303c.

The arrangement of Fig. 2 optionally further comprises a valve V24, which is arranged in the dialysis liquid inlet line 104 upstream of the blood filter 303 but downstream of a pressure meter PS5. It optionally further comprises a valve V25, which is arranged in the dialysate outlet line 102, downstream of the blood filter 303, but upstream of a further pressure meter PS4.

The arrangement in Fig. 2 comprises an optional detector 315 for detecting air and/or blood. The arrangement in Fig. 2 further encompasses at least one or more pressure sensors, here the pressure meter or pressure sensor PS1 (here exemplarily, upstream of the blood pump 101) and PS2 (here exemplarily, downstream of the blood pump 101, it measures the pressure upstream of the blood filter 303 ("prehemofilter")) at the points shown in Fig. 2. Similarly, a further venous pressure meter PS3 is also provided, for example downstream of the venous blood chamber 329. Further pressure sensors can be provided. Of the aforementioned pressure meters PS1 and PS3, only one is mandatory, the remaining others can each be provided optionally.

An optional single-needle chamber 317 is used in Fig. 2 as a buffer and/or compensating reservoir in a single-needle procedure in which the patient is connected to the extracorporeal blood circuit 300 using only one of the two blood lines 301, 305.

The arrangement of Fig. 2 also comprises an optional detector 319 for detecting air bubbles and/or blood.

An optional addition site 325 for Heparin or for other anticoagulants may be provided.

On the left in Fig. 2, is shown an optional mixing device 163, which provides a predetermined mixture for the respective solution from the containers A (for A-concentrate via concentrate supply 166) and B (for B-concentrate via concentrate supply 168) for use by the blood treatment apparatus 100. The solution contains water from a water source 155 (on-line, e.g. as reverse osmosis water or from bags) which is heated, for example, in an optional heating device 162.

An optional pump 171, which can be referred to as a concentrate pump or a sodium pump, is fluidically connected to the mixing device 163 and a source of sodium, for example the container A, and/or conveys out of it. An optional pump 173, associated with container B, e.g. for bicarbonate, can also be seen.

Furthermore, Fig. 2 shows a waste outlet 153 for the effluent. An optional heat exchanger 157 and a first flow pump 159, which is suitable for de-gassing, complete the arrangement shown.

The pressure sensor PS4 downstream of the blood filter 303 on the water side, but preferably upstream the ultrafiltration pump 131 in the dialysate outlet line 102 may be provided for measuring the filtrate pressure or membrane pressure of the blood filter 303.

Blood that leaves the blood filter 303 flows through an optional venous blood chamber 329, which may comprise a de-aeration device 318 and may be in fluid communication with the pressure meter PS3.

The exemplary arrangement shown in Fig. 2 comprises the control device 150 which may be a closed-loop control device. It may be in a wired or wireless signal connection with any of the components mentioned herein - especially or in particular with the blood pump 101 - to control or regulate the blood treatment apparatus 100

By using the device for on-line mixing of the dialysis liquid, a variation of its sodium content, controlled by the control device 150, is possible within certain limits. For this purpose, in particular the measurement values determined by the conductivity sensors 163a, 163b may be taken into account. Should an adjustment of the sodium content of the dialysis liquid (sodium concentration) or of the substitute fluid turn out to be necessary or desired, this can be done by adjusting the conveying rate of the sodium pump 171.

In addition, the blood treatment apparatus 100 comprises devices for conveying fresh dialysis liquid and dialysate. So for example, valve V24 may be provided between the first flow pump 159 and the blood filter 303, which opens or closes the inlet to the blood filter 303 on the inlet side. A second, optional flow pump 169 is provided, for example, downstream of the blood filter 303, which conveys dialysate to the waste outlet 153. The valve V25 can be provided between the blood filter 303 and the second flow pump 169, which opens or closes the drain on the outlet side.

Furthermore, the blood treatment apparatus 100 optionally comprises a device 161 for balancing the flow flowing into and out of the dialyzer 303 on the machine side. The device 161 for balancing is preferably arranged in a line section between the first flow pump 159 and the second flow pump 169.

The blood treatment apparatus 100 further comprises devices, such as the ultrafiltration pump 131, for the precise removal of a volume of liquid from the balanced circuit, as predetermined by the user and/or by the control device 150.

Sensors such as the optional conductivity sensors 163a, 163b serve to determine the conductivity, which in some embodiments is temperature-compensated, as well as the liquid flow upstream and downstream of the dialyzer 303.

Temperature sensors 165a, 165b may be provided as one or a plurality thereof. Temperature values supplied by them may be used to determine a temperature-compensated conductivity.

A leakage sensor 167 is optionally provided. Alternatively, it may also be provided at a different location.

Further flow pumps in addition to or alternatively to e.g. the one with the reference numeral 169 may also be provided.

A number of optional valves are each denoted with V in Fig. 2, bypass valves with VB.

Based on the measurement values of the above-mentioned, optional sensors, the control device 150 determines in some embodiments the electrolyte and/or fluid balance.

Filters F1 and F2 can be provided connected in series.

Even when using non-pure water, the filter F1 exemplarily serves herein to generate sufficiently pure dialysis liquid by the mixing device 163, which then flows through the blood filter 303, e.g. using the countercurrent principle.

The filter F2 exemplarily serves herein to generate sterile or sufficiently filtered substitute fluid from the sufficiently pure dialysis liquid leaving the first filter F1, by filtering e.g. pyrogenic substances. This substitute fluid may then be safely added to the extracorporeally flowing blood of the patient and thus ultimately to the patient's body.

The blood treatment apparatus 100 is optionally shown in Fig. 2 as a device for hemo(dia)filtration. However, hemodialysis devices, as well as blood treatment apparatuses which function differently, are also covered by the present invention, although not specifically represented in a figure.

The arrows shown in Fig. 2 generally indicate the direction of flow in each case.

The present invention is not limited to the embodiment described above, which is for illustrative purposes only.

### Reference Numerals

- 1: system
- 5: computer

- 100: medical treatment apparatus
- 101: blood pump
- 102: dialysate outlet line
- 104: dialysis liquid inlet line
- 105: substitute fluid line
- 107: pre-dilution valve
- 107a: line leading or belonging to the pre-dilution valve
- 109: post-dilution valve
- 109a: line leading to or belonging to the post-dilution valve
- 111: substitute fluid pump
- 131: ultrafiltration pump

- 150: control device
- 153: waste outlet
- 155: water source
- 157: heat exchanger
- 159: first flow pump
- 161: balancing device
- 162: heating device
- 163: mixing device
- 163a: conductivity sensor
- 163b: conductivity sensor
- 165a: temperature sensor
- 165b: temperature sensor
- 166: concentrate supply
- 167: leakage sensor
- 168: concentrate supply
- 169: second flow pump
- 171: pump; sodium pump
- 173: pump; bicarbonate pump

- 300: extracorporeal blood circuit
- 301: arterial blood line
- 302: (first) tubing clamp
- 303: blood filter or dialyzer
- 303a: dialysis liquid chamber
- 303b: blood chamber
- 303c: semi-permeable membrane
- 305: venous blood line
- 306: (second) tubing clamp
- 315: detector
- 317: single-needle chamber
- 318: de-aeration device
- 319: detector
- 325: addition site for Heparin, anticoagulant
- 329: venous blood chamber (optional)

- 1010: medical examination device
- 1030: device for additional input
- 1050: output device
- 1060: alarm device
- 1070: computer readable medium; clinical database; storage device
- 1100: computer program; advanced analytics based algorithms
- 1200: modular prognostic layer
- 1300: utility maximization function
- 1400: further data, additional relevant functions
- 1500: utility maximization layer
- F1: filter
- F2: filter

- A: container for A-concentrate; sodium
- B: container for B-concentrate; bicarbonate

- C: clinician

- Pa: patient

- P: pressure measurement sites
- PS1: arterial pressure meter (optional)
- PS2: arterial pressure meter (optional)
- PS3: pressure meter (optional)
- PS4: second pressure meter for measuring the filtrate pressure (optional)
- PS5: pressure meter for measuring the pressure in the dialysis liquid inlet line

- V: valves
- V24: valve
- V25: valve
- VB: bypass valve

- Y: Y-connector

## Claims

1. A system (1) for guiding a clinician in assessing the state and/or prognosis of a patient's vascular access, comprising
- a programmable computer (5);
- an input device (100, 1010, 1030, 1070);
- an output device (1050)
the input device (100, 1010, 1030, 1070) being configured for receiving or collecting input data related to the patient's vascular access;
the computer (5) being configured to assess, in an assessment step, the state of the patient's vascular access and/or to provide a prognosis with respect to the patient's vascular access based on the input data;
the computer (5) further being configured for outputting the result of said assessment step via the output device (1050).

2. The system (1) according to claim 1, the computer (5) further being configured to, in the assessment step, assess the state of the patient's vascular access and/or to provide a prognosis with respect to the patient's vascular access based on at least one predefined criterion.

3. The system (1) according to claim 1 or 2 wherein the input data is or comprises patient examination or diagnosis data, in particular longitudinal patient data, in particular with respect to the patient's vascular access, physiological patient data, and/or medical device data.

4. The system (1) according to claim 3, wherein the examination or diagnosis data is selected from the group consisting of or comprising images of the vascular access, audios of the vascular access, pulse wave data from pulse-wave imaging based on ultrasound elasticity imaging for the spatial-temporal mapping of the pulse wave-induced arterial wall motion, at least one clinician's assessment, and the patient's report.

5. The system (1) according to any one of the preceding claims, wherein the computer (5) is configured to assess the input data, in the assessment step, based on advanced analytics.

6. The system (1) according claim 5, wherein the advanced analytics comprise algorithms based on neural networks, machine learning and/or deep learning, or consists thereof.

7. The system (1) according to any one of the preceding claims, wherein the computer (5) is configured to assign or allot, in the assessment step, a diagnostic and/or prognostic value to the input data, e.g. to the patient examination or diagnosis data, physiological patient data, and/or medical device data, individually, or to the input data in general.

8. The system (1) according to any one of the preceding claims, wherein
- the computer (5) is configured to consider, in the assessment step, the medical resources or devices available.

9. The system (1) according to claim 8, wherein the computer (5) is configured to allot, in the assessment step, a benefit value to the individual medical resources or devices available, in particular with respect to the input or the diagnostic and/or prognostic value(s).

10. The system (1) according to any one of the preceding claims, wherein
- the computer (5) is configured to use, in the assessment step, a utility maximization layer and/or utility maximization function (1300).

11. The system (1) according to any one of the preceding claims, wherein
- the computer (5) is configured to output the result of said assessment step on the output device (1050), wherein the output may comprise both the diagnostic and/or prognostic value(s) allotted to the input data and the benefit values allotted to the individual medical resources or devices available.

12. The system (1) according to any one of the preceding claims, the computer (5) being configured to repeatedly carry out the assessment step or sub-steps thereof in particularly automatically, in particularly if, when or each time additional input data related to the patient's vascular access is received or collected by the at least one input device (100, 1010, 1030, 1070).

13. The system (1) according to any one of the preceding claims, further comprising
- a medical treatment apparatus (100), the medical treatment apparatus (100) being in signal communication with the input device (100, 1010, 1030, 1070) or the computer (5), or prepared therefor, the computer (5) being programmed to use data provided by the medical treatment apparatus (100) in the assessment step.

14. The system (1) according to any one of the preceding claims, further comprising
- at least one medical examination device (1010), the medical examination device (1010) being in signal communication with the input device (100, 1010, 1030, 1070) or the computer (10), or prepared therefor, the computer (5) being programmed to use data provided by the medical examination device (1010) in the assessment step.

15. A method for guiding a clinician in assessing the state and/or prognosis of a patient's vascular access, comprising the steps:
- providing a system (1) according to the present invention;
- receiving or collecting input data related to the patient's vascular access
- inputting said data into the computer (5) via the input device (100, 1010, 1030, 1070);
- assessing, using the computer (5) in an assessment step, the state of the patient's vascular access and/or providing a prognosis with respect to the patient's vascular access based on the input data;
- outputting, via the computer (5), the result of said assessment step via the output device (1050).
